# EUROPÄISCHE PATENTSCHRIFT

(11) **EP 2 705 819 B1**
(45) Veröffentlichungstag und Bekanntmachung des Hinweises auf die Patenterteilung: **02.11.2016**
(21) Anmeldenummer: 13182741.2
(22) Anmeldetag: 03.09.2013
(51) Int. Cl.: A61G 12/00, A61G 15/14, A61G 10/00, A61B 90/00

(54) **Medizinischer Arbeitsplatz**
Medical workstation
Poste de travail médical

(30) Priorität: 05.09.2012 DE 102012108263
(43) Veröffentlichungstag der Anmeldung: 12.03.2014
(73) Patentinhaber: Karl Storz GmbH & Co. KG, 78532 Tuttlingen (DE)
(72) Erfinder: Abri, Omid, 14129 Berlin (DE); Karge, Svenia, 12163 Berlin (DE); Karge, Thorsten, 12163 Berlin (DE); Verkin, Julian, 16540 Hohen Neuendorf (DE)

(56) Entgegenhaltungen:
- WO-A1-02/096340
- WO-A2-02/096335
- DE-A1- 3 541 017
- DE-A1-102007 051 038
- DE-U- 7 132 009
- DE-U1- 8 526 645
- US-A- 4 738 369
- US-A1- 2006 010 799

## Beschreibung

Die vorliegende Erfindung ist auf einen medizinischen Arbeitsplatz bezogen, insbesondere auf einen medizinischen Arbeitsplatz in einer Arztpraxis.

Zur medizinisch, ergonomisch und ökonomisch optimalen Gestaltung von Operationssälen und zur Integration von deren Funktionen existieren weit entwickelte Konzepte und komplexe technische Lösungsansätze, die sich bereits bewähren. Im Vergleich zu einem Operationssaal ist ein medizinischer Arbeitsplatz in einer Arztpraxis in der Regel Schauplatz einer deutlich geringeren Vielfalt medizinischer Vorgänge, die überdies deutlich weniger komplex sind. Auch die Gestaltung eines medizinischen Arbeitsplatzes in einer Arztpraxis unter medizinischen, ergonomischen und ökonomischen Gesichtspunkten ist jedoch in keiner Weise trivial.

Es erstaunt, dass die überwiegende Mehrzahl medizinischer Arbeitsplätze in Arztpraxen weiterhin kundenspezifisch und individuell entwickelt und gefertigt werden. Dabei wird das Rad immer wieder neu erfunden, bereits erworbene Erkenntnisse und Erfahrungen werden nicht systematisch und umfänglich erfasst und ausgewertet, um in nachfolgende Entwicklungen einzufließen. Es resultieren hochgradig individuelle medizinische Arbeitsplätze, in denen die ästhetischen Vorstellungen der Ärztinnen und Ärzte und der ausführenden Handwerksbetriebe sichtbar sind, die jedoch in vielen Fällen weder medizinisch noch ergonomisch noch ökonomisch einem Optimum nahekommen.

Die vorherrschende individuelle Fertigung medizinischer Arbeitsplätze schließt sinnvolle, jedoch technisch komplexe Lösungen in der Regel aus, da deren Entwicklung und Realisierung für Einzelstücke unwirtschaftlich ist. Individuell gestaltete medizinische Arbeitsplätze sind ferner in der Regel nicht so gestaltet, dass spätere Veränderungen oder Ergänzungen mit geringem Aufwand möglich wären.

Ausgehend von dieser Situation hat es sich die Anmelderin zur Aufgabe gemacht, Konzepte, Komponenten und technische Lösungen zu entwickeln, die gleichzeitig unter medizinischen, ergonomischen und ökonomischen Aspekten deutliche Verbesserungen, eine Kostenreduktion durch Serienfertigung und eine Individualisierung im sinnvollen und von Ärztinnen und Ärzten geforderten Maß ermöglichen. Die Übernahme von Konzepten, die für Operationssäle entwickelt wurden, ist dabei aufgrund deutlich anderer Anforderungen allenfalls in beschränktem Maße möglich.

In FR 2.050.560 ist eine Funktionseinheit für einen Zahnarzt beschrieben. Im Innenraum eines zylindrischen oder halbringförmigen Möbels ist ein Platz für medizinisches Personal vorgesehen.

In DE 2 141 991 ist eine Dentaleinrichtung für die zahnärztliche Praxis beschrieben. Auf einer kreisförmigen Schrankeinheit mit kreisförmiger Front ist eine Laufschiene für einen Instrumentenwagen angeordnet. Am Mittelpunkt der kreisförmigen Schrankwand ist die für Patienten vorgesehene Position angeordnet.

In DE 1 947 803 ist eine Vorrichtung mit augenärztlichen Untersuchungsgeräten beschrieben. Eine Arbeitsplatte weist die Form eines Kreisringstücks auf, in dessen Mitte ein Patientenstuhl angeordnet ist.

Im deutschen Gebrauchsmuster 7132009 ist eine Dentaleinrichtung für die zahnärztliche Praxis beschrieben. Auf einer kreisförmig um den Behandlungsstuhl angeordneten Schrankwand ist ein Gleis angeordnet. Auf dem Gleis ist ein Wagen für Instrumente bewegbar. Am Mittelpunkt der kreisförmigen Schrankwand ist die für Patienten vorgesehene Position angeordnet.

In FR 2 693 103 ist ein Möbel für eine zahnmedizinische Praxis beschrieben. Die Front des halbkreisförmigen Möbels ist zylindrisch mit einer vertikalen Symmetrieachse. In der Mitte eines halbkreisförmigen Möbels ist ein Stuhl für einen Patienten angeordnet.

In DE 198 07 242 A1 ist ein medizinisch-technischer Systemarbeitsplatz beschrieben. Eine Darreichungseinheit für medizinische Geräte ist auf Schienen einer Patientenlagerungsplatte verschiebbar angeordnet.

In DE 198 07 243 A1 ist ein medizinischer Arbeitsplatz beschrieben. Ein Gerätecenter für chirurgische und diagnostische Geräte ist auf Schienen geführt, um eine kreisförmige Verstellung zu ermöglichen.

In DE 200 02 305 U1 ist eine modulare Möbelanordnung für Arztpraxen beschrieben. Mehrere Module sind U-förmig um eine Patientenliege herum angeordnet.

In DE 295 05 059 ist eine Behandlungseinrichtung für eine Arzt-/Zahnarztpraxis beschrieben. Ein Gerätetablett und eine Instrumentenplatte sind schwenkbar gelagert.

In DE 296 00 647 ist eine Arbeitsplatte beschrieben, unter der Führungs- und Laufschienen angebracht sind, über die Möbelelemente seitlich verfahren werden können.

Im deutschen Gebrauchsmuster 7123181 ist eine Dentaleinrichtung für eine zahnärztliche Praxis beschrieben, die eine Bohr- und Behandlungseinheit in Gestalt eines fahrbaren Wagens umfasst. Die für medizinisches Personal und Patienten vorgesehenen Positionen sind innerhalb einer rechteckig U-förmigen Anordnung von Schrankeinheiten angeordnet.

In DE 2 220 068 ist ein zahnärztlicher Geräteschrank beschrieben, dessen Grundriss U-förmig ist.

In US 2007/0176060 (später veröffentlicht als US 7,644,898) ist ein medizinischer Ausleger mit bewegbaren Armen zum Halten von Videoanzeigen beschrieben.

Eine Aufgabe der vorliegenden Erfindung besteht darin, einen verbesserten medizinischen Arbeitsplatz zu schaffen.

Diese Aufgabe wird durch die Gegenstände der unabhängigen Ansprüche gelöst.

Weiterbildungen sind in den abhängigen Ansprüchen angegeben.

Ein medizinischer Arbeitsplatz umfasst Staumöbeln zur Aufnahme medizinischer Instrumente oder Utensilien und eine Transportschieneneinrichtung zum Halten eines entlang der Transportschieneneinrichtung verfahrbaren Instrumententrägers, wobei die Transportschieneneinrichtung einen Beladebereich, einen Entladebereich und zwischen dem Beladebereich und dem Entladebereich einen Verwendungsbereich aufweist, wobei der Verwendungsbereich in einem Raumbereich über den Staumöbeln angeordnet ist.

Der medizinische Arbeitsplatz ist insbesondere ein medizinischer Arbeitsplatz in einer Arztpraxis einer niedergelassenen Ärztin oder eines niedergelassenen Arztes, in einem Ärztehaus, in der Ambulanz einer Klinik. Der medizinische Arbeitsplatz ist jedoch insbesondere kein Operationssaal, in dem die Anordnung von Staumöbeln schon aufgrund anderer Abläufe und der Gegenwart assistierenden medizinischen Personals, das beispielsweise Instrumente reicht und entgegen nimmt, in der Regel irrelevant ist. Auch in weiteren, unten beschriebenen Eigenschaften und Merkmalen kann sich der medizinische Arbeitsplatz von einem Operationssaal sehr deutlich unterscheiden.

Die für medizinisches Personal, insbesondere eine Ärztin oder einen Arzt, während einer medizinischen Tätigkeit an einem Patienten vorgesehene Position ist insbesondere durch einen Sitzplatz für das medizinische Personal auf einem Hocker oder einem anderen Sitzmöbel definiert. Alternativ kann die für medizinisches Personal während einer medizinischen Tätigkeit an einem Patienten vorgesehene Position ein für das medizinische Personal vorgesehener Standort sein.

Die Staumöbel sind zur Aufnahme von medizinischen Instrumenten, Medikamenten, Verbrauchsmaterial und/oder anderen Utensilien vorgesehen und ausgebildet. Ferner können die Staumöbel zur Aufnahme von verschmutzten oder unsterilen Instrumenten nach dem Gebrauch sowie von Abfällen ausgebildet sein. Neben oder zwischen den Staumöbeln können medizinische Geräte im gleichen Kreisbogen angeordnet sein. Insbesondere können schrankförmige Geräte zur Bereitstellung von Gas, Licht, mechanischer oder elektrischer Leistung für medizinische Instrumente zusammen mit den Staumöbeln einen horizontalen Kreisbogen bilden.

Bei einem medizinischen Arbeitsplatz, wie er hier beschrieben ist, sind die Staumöbel insbesondere kreisbogenförmig um die für medizinisches Personal vorgesehene Position angeordnet.

Ein medizinischer Arbeitsplatz umfasst einen für medizinisches Personal während einer medizinischen Tätigkeit an einem Patienten vorgesehene Position und Staumöbel zur Aufnahme medizinischer Instrumente oder Utensilien, wobei die Staumöbel kreisbogenförmig um die für medizinisches Personal vorgesehene Position angeordnet sind.

Die kreisbogenförmige Anordnung von Staumöbeln und optional von medizinischen Geräten ermöglicht eine gleich gute Erreichbarkeit aller Staumöbel und Geräte von der für das medizinische Personal vorgesehenen Position aus. Die Staumöbel können rechteckige Grundrisse aufweisen. Hinsichtlich ästhetischer Aspekte und der Vermeidung von toten Winkeln, die schwer zu reinigen sind, können jedoch Staumöbel mit trapezförmigen Grundrissen und mit Grundrissen in der Gestalt von Segmenten eines Kreisbogens vorteilhaft sein. Der Entwurf, vor allem aber die Fertigung von Staumöbeln mit trapezförmigen Grundrissen und insbesondere mit Grundrissen in der Form von Kreisbogensegmenten sind jedoch deutlich aufwendiger als Entwurf und Fertigung herkömmlicher quaderförmiger Staumöbel. Auch deshalb kam vermutlich bislang eine kreisbogenförmige Anordnung von Staumöbeln und gegebenenfalls Geräten offenbar nicht in Betracht.

Bei einem medizinischen Arbeitsplatz, wie er hier beschrieben ist, bilden Frontflächen der Staumöbel Bereiche einer Kreiszylindermantelfläche, deren Symmetrieachse vertikal ist, und auf deren Symmetrieachse die für medizinisches Personal vorgesehene Position liegt.

Insbesondere bilden alle oder im Wesentlichen alle vertikalen Frontflächen der Staumöbel Bereiche einer Kreiszylindermantelfläche. Die Frontflächen bilden auch dann Bereiche einer Kreiszylindermantelfläche im Sinne der vorliegenden Beschreibung, wenn sie - beispielsweise aus ästhetischen oder fertigungstechnischen Gründen - von der idealen Kreiszylindermantelform nicht wesentlich (beispielsweise nur um wenige Prozent, insbesondere höchstens 10%, des Durchmessers des Kreiszylindermantels) von der ideal Gestalt eines Kreiszylindermantels abweichen. Wenn die für medizinisches Personal vorgesehene Position durch eine Sitzfläche definiert ist, liegt die Position insbesondere dann auf der Achse des Kreiszylindermantels, wenn die Achse die Sitzfläche in deren Mitte oder an einem anderen Punkt durchstößt, wenn also der Rand der Sitzfläche die Achse des Zylindermantels umschließt.

Die Kreiszylindermantelfläche weist insbesondere einen Durchmesser zwischen 1,5 Meter und 2,0 Meter, beispielsweise 1,8 Meter auf. Dieser Durchmesser hat sich als in vielen Situationen und für viele Ärztinnen oder Ärzte ideal herausgestellt, da er einerseits einen ausreichenden Bewegungsraum zwischen den Staumöbeln und andererseits deren gute Erreichbarkeit von der für medizinisches Personal vorgesehenen Position aus gewährleisten kann.

Bei einem medizinischen Arbeitsplatz, wie er hier beschrieben ist, ist eine für einen Patienten während einer medizinischen Tätigkeit vorgesehene Position insbesondere in dem durch die Staumöbel gebildeten Kreisbogen angeordnet.

Insbesondere ist ein Sitzplatz oder eine Liege für einen Patienten innerhalb des durch die Staumöbel und optional medizinische Geräte gebildeten Kreisbogens angeordnet. Alternativ ist lediglich der relevante Bereich oder Köperteil des Patienten in dem Kreisbogen angeordnet, beispielsweise der Kopf im Falle einer zahnärztlichen oder kieferchirurgischen oder Hals-Nasen-Ohren-heilkundlichen Praxis. Wenn der Patient oder der relevante Bereich des Patienten und die Staumöbel kreisbogenförmig um die für medizinisches Personal vorgesehene Position angeordnet sind, kann das medizinische Personal den Patienten und die Staumöbel im Wesentlichen gleich gut erreichen.

Ein medizinischer Arbeitsplatz, wie er hier beschrieben ist, umfasst insbesondere ferner eine Transportschieneneinrichtung zum Halten eines entlang der Transportschieneneinrichtung verfahrbaren Instrumententrägers, wobei die Transportschieneneinrichtung einen Beladebereich und einen Entladebereich und zwischen dem Beladebereich und dem Entladebereich einen Verwendungsbereich aufweist, wobei der Verwendungsbereich in einem Raumbereich über den Staumöbeln angeordnet ist.

Die Transportschieneneinrichtung ist insbesondere horizontal angeordnet, um ein horizontales Verfahren des oder der Instrumententräger zu ermöglichen. Ein oder mehrere Instrumententräger können manuell oder motorisch entlang der Transportschieneneinrichtung verfahrbar sein. Neben einem oder mehreren Instrumententrägern können medizinische Geräte, Tabletts, Abfallbehälter und/oder weitere Einrichtungen entlang der Transportschieneneinrichtung verfahrbar sein.

Der Beladebereich und/oder der Entladebereich können jeweils in einem teilweise oder weitgehend vom medizinischen Arbeitsplatz abgetrennten Raumbereich angeordnet sein. Insbesondere können zwischen der für einen Patienten vorgesehenen Position und dem Beladebereich und/oder zwischen für einen Patienten vorgesehenen Position und dem Entladebereich Trennwände vorgesehen sein. Der Beladebereich und der Entladebereich können im gleichen oder im Wesentlichen gleichen oder in zwei aneinander angrenzenden Raumebereichen angeordnet sein. Alternativ können der Beladebereich und der Entladebereich in zwei voneinander beabstandeten Raumbereichen angeordnet sein. Dabei ist insbesondere der medizinische Arbeitsplatz zwischen dem Raumbereich, in dem der Beladebereich angeordnet ist, und dem Raumbereich, in dem der Entladebereich angeordnet ist, angeordnet.

Die Transportschieneneinrichtung ermöglicht ein Bestücken eines ersten Instrumententrägers mit sterilen Instrumenten und/oder Verbrauchsmaterialien im Beladebereich, ein Entleeren eines zweiten Instrumententrägers im Entladebereich und gleichzeitig eine Verwendung eines dritten Instrumententrägers und von Instrumenten auf dem dritten Instrumententräger im Verwendungsbereich, ohne dass diese drei Vorgänge einander behinderten. Der erforderliche zeitliche Abstand zwischen medizinischen Tätigkeiten an verschiedenen Patienten kann damit verkürzbar sein.

Bei einem medizinischen Arbeitsplatz, wie er hier beschrieben ist, ist die Transportschieneneinrichtung insbesondere in einer horizontalen Ebene über und beabstandet von den Staumöbeln angeordnet.

Bei einem medizinischen Arbeitsplatz mit einer Transportschieneneinrichtung, wie er hier beschrieben ist, ist der Verwendungsbereich insbesondere bogenförmig ausgebildet.

Insbesondere ist der Verwendungsbereich kreisbogenförmig bzw. für eine Verfahrbarkeit des Instrumententrägers entlang eines Kreisbogens ausgebildet, wobei der Krümmungsmittelpunkt an oder vertikal über der für medizinisches Personal während einer medizinischen Tätigkeit an einem Patienten vorgesehenen Position angeordnet ist.

Bei einer bogenförmigen und insbesondere bei einer kreisbogenförmigen Ausbildung des Verwendungsbereichs kann ein Instrumententräger an allen Orten an der Transportschieneneinrichtung innerhalb des Verwendungsbereichs gleich gut von der für medizinisches Personal vorgesehenen Position aus erreichbar sein.

Bei einem medizinischen Arbeitsplatz mit einer Transportschieneneinrichtung, wie er hier beschrieben ist, weist insbesondere zumindest entweder der Beladebereich oder der Entladebereich einen größeren Abstand von der für medizinisches Personal vorgesehenen Position auf als der Verwendungsbereich.

Ein medizinischer Arbeitsplatz, wie er hier beschrieben ist, umfasst insbesondere ferner eine bogenförmige Tragschieneneinrichtung zum Halten eines Geräts, das für die Verwendung durch medizinisches Personal vorgesehen und entlang der Tragschieneneinrichtung verschiebbar ist.

Die bogenförmige Tragschieneneinrichtung ist insbesondere kreisbogenförmig bzw. zum Halten eines Geräts an einem von mehreren alternativen Orten, die auf einem Kreisbogen liegen, ausgebildet. Die bogenförmige Tragschieneneinrichtung kann mehrere Geräte halten. Beispiele für durch die Tragschieneneinrichtung gehaltene Geräte sind ein Monitor bzw. Bildschirm oder eine andere Anzeigevorrichtung, ein Haltearm für ein Endoskop, für ein Exoskop, für ein Mikroskop oder für ein anderes medizinisches Instrument oder Gerät, ein Haltearm für eine Benutzerschnittstelle eines medizinischen Geräts.

An der Tragschieneneinrichtung können das oder die Geräte so gehalten werden, dass sie ohne Verwendung von Werkzeug und mit geringem Kraftaufwand entlang der Tragschieneneinrichtung verschoben werden können. Dazu können zwischen einem Gerät und der Tragschieneneinrichtung jeweils ein oder mehrere Gleit- und/oder Rolllager vorgesehen sein, beispielsweise an einer Laufkatze. Alternativ können das oder die Geräte derart an der Tragschieneneinrichtung gehalten sein, dass lediglich unter Verwendung von Werkzeug und/oder mit hohem Kraftaufwand die Positionen der Geräte verändert werden können.

Die bogenförmige Tragschieneneinrichtung kann eine individuelle Anpassung der Position oder Positionen von Geräten an die Erfordernisse unterschiedlicher medizinischer Tätigkeit und/oder an die Bedürfnisse und Gewohnheiten verschiedenen medizinischen Personals ermöglichen.

Die Tragschieneneinrichtung ist insbesondere in einer horizontalen Ebene über und beabstandet von den Staumöbeln angeordnet.

Bei einem medizinischen Arbeitsplatz mit einer Tragschieneneinrichtung, wie er hier beschrieben ist, weist die Tragschieneneinrichtung insbesondere zumindest einen kreisbogenförmigen Abschnitt mit einem Mittelpunkt an oder über der für medizinisches Personal vorgesehenen Position auf.

Eine kreisbogenförmige Ausbildung der Tragschieneneinrichtung ist nicht nur in Kombination mit der kreisbogenförmigen Anordnung der Staumöbel ästhetisch vorteilhaft, sondern schafft vor allem auch eine Möglichkeit einer weitgehenden Anpassung des medizinischen Arbeitsplatzes an unterschiedliche Erfordernisse, Gewohnheiten und Bedürfnisse.

Bei einem medizinischen Arbeitsplatz mit einer Tragschieneneinrichtung, wie er hier beschrieben ist, weist die Tragschieneneinrichtung insbesondere einen Kanal zur Aufnahme eines Kabel oder eines Schlauchs auf.

Der Kanal kann einen durchgehend offenen oder einen abschnittsweise geschlossenen Querschnitt aufweisen und zur Aufnahme von einem oder mehreren Kabeln und/oder Schläuchen ausgebildet sein. Die Anordnung von Kabeln und/oder Schläuchen zur Versorgung von durch die Tragschieneneinrichtung gehaltenen Geräten mit elektrischen und/oder optischen Signalen, Leistung, Druckluft oder anderen Fluiden in dem Kanal kann die Installation der Geräte vereinfachen und einen sauberen ordentlichen Eindruck fördern.

Bei einem medizinischen Arbeitsplatz mit einer Tragschieneneinrichtung mit einem Kanal, wie er hier beschrieben ist, weist die Tragschieneneinrichtung insbesondere einen nach oben offenen U-förmigen Querschnitt auf, wobei das Innere des Querschnitts der Tragschieneneinrichtung den Kanal bildet.

Bei einem medizinischen Arbeitsplatz, wie er hier beschrieben ist, weist ein Staumöbel insbesondere einen Korpus mit einer Bodenplatte in der Form eines Segments eines Kreisrings und nicht-parallelen Seitenwänden und in dem Korpus einen Einsatz zum Aufnehmen eines Gegenstands auf, wobei der Einsatz parallele Seitenwände aufweist.

Ein Segment eines Kreisrings weist einen Rand mit zwei kreisbogenförmigen Abschnitten und zwei geraden Abschnitten auf, wobei die Kreisbögen Teile zweier konzentrischer Kreise und die Geraden Radien dieser Kreise sind. Der Korpus kann ferner eine gekrümmte Rückwand aufweisen, insbesondere eine Rückwand in Gestalt eines Ausschnitts eines Kreiszylindermantels. Der Einsatz umfasst insbesondere ein oder mehrere offene Fächer, ein oder mehrere Schubladeneinheiten mit jeweils einer oder mehreren Schubladen, einen oder mehrere Abwürfe zum Ablegen von unsterilen Instrumenten oder verwendeten Verbrauchsmaterialien und/oder ein oder mehrere Besteckfacheinheiten. Der Einsatz kann zur Aufnahme mehrerer Gegenstände unterschiedlicher Größe, Funktion und sonstiger Eigenschaften ausgebildet sein.

Bei einem medizinischen Arbeitsplatz mit einem Korpus und einem Einsatz in dem Korpus, wie er hier beschrieben ist, umfasst der Einsatz insbesondere einen Auszug.

### Kurzbeschreibung der Figuren

Nachfolgend werden Ausführungsformen anhand der beigefügten Figuren näher erläutert. Es zeigen:
- Figur 1: eine schematische Darstellung eines medizinischen Arbeitsplatzes;
- Figur 2: eine schematische Darstellung eines weiteren medizinischen Arbeitsplatzes;
- Figur 3: eine schematische Darstellung eines weiteren medizinischen Arbeitsplatzes;
- Figur 4: eine schematische Darstellung eines weiteren medizinischen Arbeitsplatzes;
- Figur 5: eine weitere schematische Darstellung des medizinischen Arbeitsplatzes aus Figur 4;
- Figur 6: eine weitere schematische Darstellung des medizinischen Arbeitsplatzes aus den Figuren 4 und 5;
- Figur 7: eine schematische Darstellung einer Tragschieneneinrichtung;
- Figur 8: eine schematische Darstellung eines Geräts;
- Figur 9: eine schematische Darstellung eines Staumöbels;
- Figur 10: eine weitere schematische Darstellung des Staumöbels aus Figur 9;
- Figur 11: eine schematische Darstellung von Bestandteilen des Staumöbels aus den Figuren 9 und 10;
- Figur 12: eine weitere schematische Darstellung des Staumöbels aus den Figuren 9 bis 11;
- Figur 13: eine schematische Darstellung eines Einsatzes für ein Staumöbel;
- Figur 14: eine schematische Darstellung eines weiteren Einsatzes für ein Staumöbel;
- Figur 15: eine schematische Darstellung eines weiteren Einsatzes für ein Staumöbel;
- Figur 16: eine schematische Darstellung eines weiteren Staumöbels;
- Figur 17: eine schematische Darstellung eines schrankförmigen Geräts;
- Figur 18: eine schematische Darstellung eines weiteren Staumöbels.

### Beschreibung der Ausführungsformen

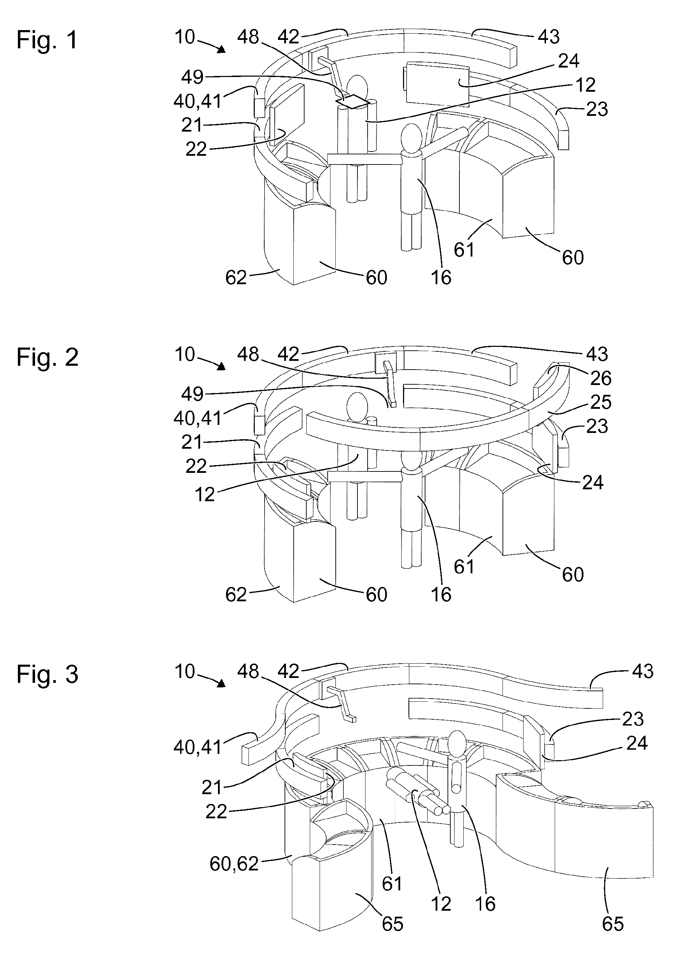

Figur 1 zeigt eine schematische axonometrische Darstellung eines medizinischen Arbeitsplatzes 10 für einen Patienten 12 und medizinisches Personal 16, insbesondere eine Ärztin oder einen Arzt. Der Patient 12 und das medizinische Personal 16 sind jeweils an den für sie vorgesehenen Positionen dargestellt. Abweichend von der Darstellung in Figur 1 kann der Patient 12 sitzen oder liegen und/oder das medizinische Personal 16 sitzen. Nachfolgend wird das Bezugszeichen 12 sowohl für den Patienten als auch für die während einer medizinischen Tätigkeit des medizinischen Personals an dem Patienten vorgesehenen Positionen des Patienten verwendet. Ferner wird das Bezugszeichen 16 sowohl für das medizinische Personal als auch für die während einer medizinischen Tätigkeit des medizinischen Personals an dem Patienten vorgesehenen Positionen des medizinischen Personals verwendet.

Der medizinische Arbeitsplatz 10 umfasst eine kreisbogenförmige erste Tragschiene 21 und eine kreisbogenförmige zweite Tragschiene 23. Die kreisbogenförmigen Tragschienen 21, 23 sind so angeordnet, dass ihre Krümmungsmittelpunkte sich an oder vertikal über der für medizinisches Personal 16 vorgesehenen Position befinden. Die Tragschieneneinrichtungen 21, 23 sind beiderseits der für den Patienten 12 vorgesehenen Position angeordnet. Die Tragschieneneinrichtungen 21, 23 sind in einer horizontalen Ebene angeordnet.

Ein erster Bildschirm 22 ist von der ersten Tragschiene 21 gehalten und entlang der ersten Tragschiene 21 verschiebbar oder verfahrbar. Ein zweiter Bildschirm 24 ist von der zweiten Tragschiene 23 gehalten und entlang dieser verschiebbar oder verfahrbar. An jeder der Tragschieneneinrichtungen 21, 23 können alternativ oder zusätzlich weitere Geräte, insbesondere medizinische Geräte gehalten und verfahrbar sein.

Zwischen den Tragschienen 21, 23 einerseits und den Bildschirmen 22, 24 oder anderen an den Tragschienen 21, 23 gehaltenen Geräten andererseits können Gleit- oder Rolllager vorgesehen sein, die ein Verschieben oder Verfahren mit geringem Kraftaufwand ermöglichen. Alternativ können die Bildschirme 22, 24 und/oder andere Geräte derart an den Tragschienen 21, 23 gehalten sein, dass sie jeweils nur mit erheblichem Kraftaufwand und/oder unter Verwendung von Werkzeug entlang den Tragschienen 21, 23 verschiebbar sind. Ferner können die Tragschienen 21, 23 derart ausgestaltet sein, dass die Bildschirme 22, 24 und/oder andere Geräte lediglich an einer vorbestimmten Anzahl von diskreten und voneinander beabstandeten Positionen angeordnet, insbesondere eingehängt werden können.

Ferner umfasst der medizinische Arbeitsplatz 10 eine Transportschieneneinrichtung 40 mit einem Beladebereich 41 links des Patienten 12 (vom medizinischen Personal 16 aus gesehen), einem Verwendungsbereich 42 in der Nähe des Patienten 12 und einem Entladebereich 43 rechts des Patienten 12. Beladebereich 41 und Entladebereich 43 können vertauscht sein. Alternativ können beide Bereiche 41, 43 sowohl zum Beladen als auch zum Entladen vorgesehen sein. Die Transportschieneneinrichtung 40 ist in einer horizontalen Ebene über den Tragschieneneinrichtungen 21, 23 angeordnet.

Ein Haltearm 48 für einen Instrumententräger 49 ist von der Tragschieneneinrichtung 40 gehalten und entlang dieser verfahrbar. Dazu sind insbesondere ein oder mehr Gleit- und/oder Rolllager zwischen der Transportschieneneinrichtung 40 und dem Haltearm 48 vorgesehen.

Der in dem gezeigten Beispiel tablettförmige Instrumententräger 49 kann beispielsweise im Beladebereich 41 mit sterilen Instrumenten und/oder Verbrauchsmaterial beladen und bei Bedarf in den Verwendungsbereich 42 nahe dem Patienten 12 verschoben werden. Nach der Verwendung von auf dem Instrumententräger 49 am Haltearm 48 bereitgestellten Instrumenten im Rahmen einer medizinischen Tätigkeit kann der Haltearm 48 mit dem Instrumententräger 49 in den Entladebereich 43 der Transportschieneneinrichtung verfahren werden. Da der Beladebereich 41 und der Entladebereich 43 vom Verwendungsbereich 42 und vom Patienten 12 beabstandet sind, können gleichzeitig im Beladebereich 41 ein erster Instrumententräger beladen bzw. bestückt und im Entladebereich 43 ein zweiter Instrumententräger entladen bzw. entleert werden, ohne eine medizinische Tätigkeit des medizinischen Personals 16 am Patienten 12 (insbesondere unter Verwendung von Instrumenten, die auf einem dritten Instrumententräger im Verwendungsbereich 42 bereitgestellt sind) zu beeinträchtigen.

Im Wesentlichen unter den horizontalen Ebenen, in denen die Tragschieneneinrichtungen 21, 23 und die Transportschieneneinrichtung 40 angeordnet sind, sind Staumöbel 60 vorgesehen. Die Staumöbel 60 sind kreisbogenförmig angeordnet. Die für einen Patienten vorgesehene Position 12 ist ebenfalls in dem durch die Staumöbel 60 gebildeten Kreisbogen angeordnet. Zusätzlich zu den Staumöbeln 60 können Geräte, insbesondere schrankförmige Geräte in demselben Kreisbogen angeordnet sein. Der Mittelpunkt des Kreisbogens liegt an der für das medizinische Personal vorgesehenen Position 16.

Das einzelne Staumöbel 60 weist insbesondere die Gestalt eines Segments eines Kreisbogens auf. Insbesondere weist das einzelne Staumöbel 60 eine Frontfläche 61 und eine Rückwand 62 in Gestalt zweier Ausschnitte von Kreiszylindermänteln auf. Die Staumöbel 60 sind so angeordnet, dass die für medizinisches Personal vorgesehene Position 16 auf der gemeinsamen vertikalen Symmetrieachse der Kreiszylindermäntel, deren Ausschnitte die Frontflächen 61 und die Rückwände 62 bilden, liegt.

Figur 2 zeigt eine schematische axonometrische Darstellung eines weiteren medizinischen Arbeitsplatzes 10, der in einigen Merkmalen und Eigenschaften dem oben anhand der Figur 1 dargestellten medizinischen Arbeitsplatz ähnelt. Nachfolgend werden im Wesentlichen nur jene Merkmale und Eigenschaften beschrieben, in denen sich der medizinische Arbeitsplatz 10 von dem oben anhand der Figur 1 dargestellten unterscheidet.

Der medizinische Arbeitsplatz 10 umfasst eine dritte Tragschieneneinrichtung 25. Ein dritter Bildschirm 26 ist von der dritten Tragschieneneinrichtung 25 gehalten und entlang dieser verschiebbar oder verfahrbar. Die dritte Tragschieneneinrichtung 25 ist insbesondere so angeordnet, dass der dritte Bildschirm 26 während einer medizinischen Tätigkeit am Patienten 12 in dessen Blickfeld liegt. Um eine Abdeckung des dritten Bildschirms 26 durch das medizinische Personal 16 zu verhindern, kann der dritte Bildschirm 26 entlang der dritten Tragschieneneinrichtung 25 verschoben werden. Der dritte Bildschirm 26 ist beispielsweise zur Darstellung eines Films oder anderer Inhalte, die den Patienten 12 entspannen, verwendbar oder kann dem Patienten 12 ermöglichen, einen medizinischen Eingriff gleichzeitig mitzuverfolgen.

Zur Illustration der Verschiebbarkeit bzw. Verfahrbarkeit der Bildschirme 22, 24 entlang den Tragschienen 21, 23 sind die Bildschirme 22, 24 abweichend von der Darstellung in Figur 1 in Positionen angeordnet, die vom Patienten 12 weit beabstandet sind. Der erste Bildschirm 22 an der ersten Tragschieneneinrichtung 21 und der zweite Bildschirm 24 an der zweiten Tragschieneneinrichtung 23 werden beispielsweise dann in die in Figur 2 gezeigten Positionen verschoben, wenn sie für die momentane medizinische Tätigkeit nicht erforderlich sind.

Figur 3 zeigt eine schematische axonometrische Darstellung eines weiteren medizinischen Arbeitsplatzes 10, der in einigen Merkmalen und Eigenschaften den oben anhand der Figuren 1 und 2 dargestellten medizinischen Arbeitsplätzen ähnelt. Nachfolgend werden im Wesentlichen nur jene Merkmale und Eigenschaften beschrieben, in denen sich der medizinische Arbeitsplatz 10 von den oben anhand der Figuren 1 und 2 dargestellten unterscheidet.

Der medizinische Arbeitsplatz 10 unterscheidet sich von den oben anhand der Figuren 1 und 2 dargestellten medizinischen Arbeitsplätzen unter anderem dadurch, dass die für den Patienten 12 vorgesehene Position nicht zwischen den Staumöbeln 60 liegt und mit diesen zusammen einen Kreisbogen bildet. Stattdessen bilden die Staumöbel 60 einen vollständig oder im Wesentlichen geschlossen halbkreisförmigen Bogen, und der Patient 12 ist im Inneren dieses Bogens angeordnet.

Ferner unterscheidet sich der in Figur 3 dargestellte medizinische Arbeitsplatz 10 von den oben anhand der Figuren 1 und 2 dargestellten medizinischen Arbeitsplätzen dadurch, dass anschließend an die beiden Enden einer zentralen, im Wesentlichen halbkreisbogenförmigen Anordnung von Staumöbeln 60 sich weitere Staumöbel 65 anschließen, die umgekehrt gekrümmte bzw. nach außen gebogene Kreisbögen bilden. Diese weiteren Staumöbel 65 sind beispielsweise bei der Vor- und Nachbereitung medizinischer Tätigkeiten verwendbar.

Ferner unterscheidet sich der in Figur 3 dargestellte medizinische Arbeitsplatz 10 von den oben anhand der Figuren 1 und 2 dargestellten dadurch, dass die Tragschieneneinrichtung 40 an beiden Enden in Bögen umgekehrter Krümmung verlängert sind. Insbesondere sind der Beladebereich 41 und der Entladebereich 43 der Tragschieneneinrichtung 40 über den weiteren Staumöbeln 65 angeordnet. Entsprechend der umgekehrten Krümmung der Anordnung der weiteren Staumöbel 65 weisen der Beladebereich 41 und der Entladebereich 43 zum Verwendungsbereich 42 entgegengesetzte Krümmungen auf.

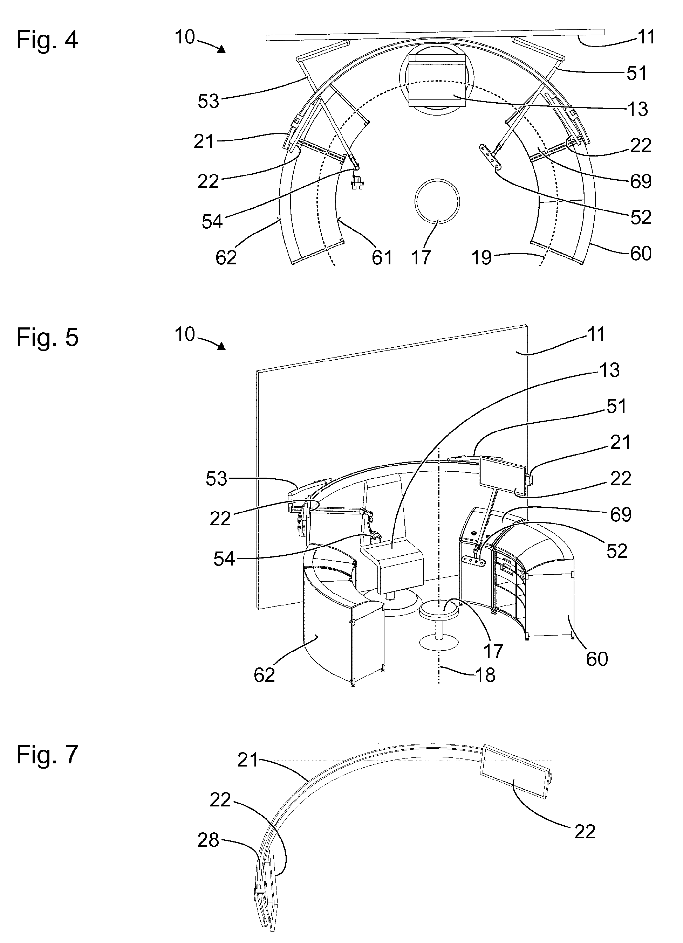

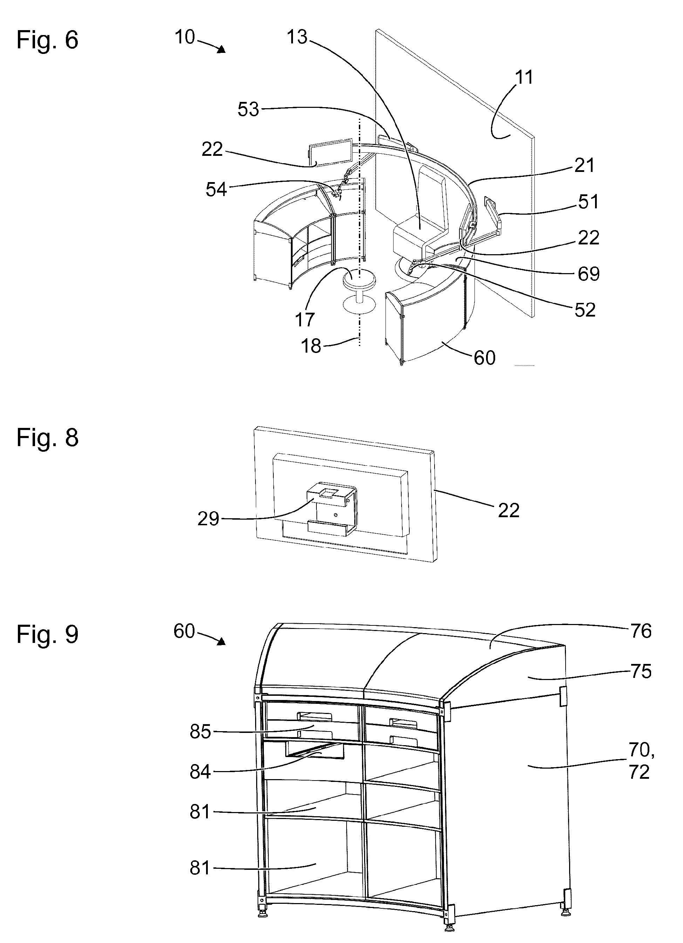

Figur 4 zeigt eine schematische Draufsicht eines weiteren medizinischen Arbeitsplatzes 10, der in einigen Merkmalen und Eigenschaften den oben anhand der Figuren 1 bis 3 dargestellten medizinischen Arbeitsplätzen ähnelt. Figur 5 zeigt eine schematische axonometrische Darstellung des medizinischen Arbeitsplatzes aus Figur 4. Figur 6 zeigt eine weitere schematische axonometrische Darstellung des medizinischen Arbeitsplatzes aus den Figuren 4 und 5. Nachfolgend werden im Wesentlichen nur jene Merkmale und Eigenschaften beschrieben, in denen sich der medizinische Arbeitsplatz 10 von den oben anhand der Figuren 1 bis 3 dargestellten unterscheidet.

Bei dem in den Figuren 4, 5 und 6 dargestellten medizinischen Arbeitsplatz 10 sind die für einen Patienten vorgesehene Position durch einen Behandlungsstuhl 13 und die für medizinisches Personal vorgesehene Position durch einen Sitzplatz 17, insbesondere einen Hocker definiert. Ähnlich wie bei den oben anhand der Figuren 1 und 2 dargestellten medizinischen Arbeitsplätzen bildet die für einen Patienten vorgesehene Position 12 zusammen mit Staumöbeln 60 einen Kreisbogen, der in der Draufsicht der Figur 4 in gestrichelter Linie angedeutet ist. In den Figuren 5 und 6 ist die vertikale Symmetrieachse 18 der kreiszylindermantelförmigen Frontflächen 61 und Rückflächen 62 der Staumöbel 60 angedeutet.

Der medizinische Arbeitsplatz 10 der Figuren 4 bis 6 unterscheidet sich von den oben anhand der Figuren 1 und 2 dargestellten medizinischen Arbeitsplätzen ferner dadurch, dass lediglich eine Tragschieneneinrichtung 21 vorgesehen ist, an der beispielhaft zwei Bildschirme 22 verschiebbar gehalten sind. Eine Transportschieneneinrichtung für einen Instrumententräger - wie anhand der Figuren 1 bis 3 dargestellt - ist nicht vorgesehen, kann jedoch ergänzt werden.

Ferner unterscheidet sich der medizinische Arbeitsplatz 10 der Figuren 4 bis 6 von den oben anhand der Figuren 1 und 2 dargestellten medizinischen Arbeitsplätzen dadurch, dass an einer Raumwand 11 Haltearm 51, 53 angelenkt sind, die Instrumententräger 52, 54 halten.

Ferner unterscheidet sich der medizinische Arbeitsplatz 10 der Figuren 4 bis 6 von den oben anhand der Figuren 1 und 2 dargestellten dadurch, dass der Kreisbogen 19 (vgl. Figur 4) nicht nur durch Staumöbel 60 und den Behandlungsstuhl 13, sondern ferner durch ein schrankförmiges medizinisches Gerät 69 gebildet wird. Das schrankförmige medizinische Gerät 69 enthält beispielsweise eine Steuereinheit, eine elektrische Leistungsversorgung, eine Lichtquelle und/oder Versorgungseinrichtungen zum Bereitstellen von Druckluft oder anderen Fluiden für zahnmedizinische Instrumente oder für ein Endoskop und/oder andere medizinische Instrumente für mikroinvasive Eingriffe.

Figur 7 zeigt eine schematische axonometrische Darstellung einer Tragschieneneinrichtung 21 mit zwei Bildschirmen 22. Die Tragschieneneinrichtung 21 ist insbesondere eine der Tragschieneneinrichtungen der medizinischen Arbeitsplätze aus den Figuren 1 bis 6.

Die Tragschieneneinrichtung 21 weist einen nach oben offenen U-förmigen Querschnitt auf, dessen Inneres einen Kabelkanal 28 bildet. Kabel zur elektrischen Leistungsversorgung der Bildschirme 22 oder anderer von der Tragschieneneinrichtung 21 gehaltener Geräte sowie elektrische und/oder optische Signalleitungen und Fluidleitungen zu den Bildschirmen 22 oder anderen Geräten können in dem Kabelkanal 28 angeordnet sein.

Figur 8 zeigt eine schematische axonometrische Darstellung der Rückseite eines Bildschirms 22. An der Rückseite des Bildschirms 22 ist eine Befestigungseinrichtung 29 vorgesehen, mittels derer der Bildschirm 22 an einer Tragschieneneinrichtung 21, 23, 25 (vgl. Figuren 1 bis 7) gehalten sein kann. Insbesondere ist die Befestigungseinrichtung 29 C-förmig ausgebildet, um die Tragschieneneinrichtung 21, 23, 25 oben und unten zu umgreifen oder zu hintergreifen. Die Materialien der Befestigungseinrichtung 29 und der Tragschieneneinrichtungen 21, 23, 25 können vorgesehen sein, um ein leichtes Gleiten entlang der Tragschieneneinrichtungen 21, 23, 25 zu ermöglichen. Alternativ sind zu diesem Zweck Gleit- oder Rolllager vorgesehen, die in Figur 8 nicht dargestellt sind.

Figur 9 zeigt eine schematische axonometrische Darstellung eines Staumöbels 60, das bei den medizinischen Arbeitsplätzen 10 aus den Figuren 1 bis 6 verwendbar ist. Das Staumöbel 60 weist einen Korpus 70 auf, von dem in Figur 9 vor allem eine große rechteckige untere Seitenwand 72 und eine kleinere obere Seitenwand 75 sowie eine obere Abdeckung 76 sichtbar sind. Der Korpus 70 weist im Wesentlichen die Gestalt eines Segments eines Kreisbogens auf, insbesondere sind die Seitenwände 72, 75 radial angeordnet.

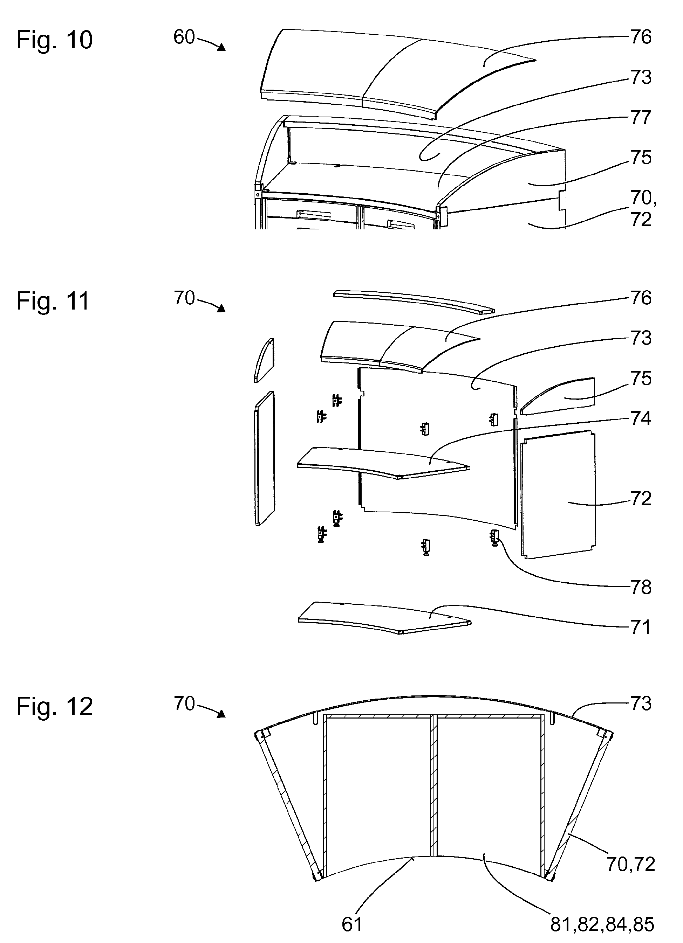

Im Korpus 70 sind mehrere Einsätze 81, 84, 85 angeordnet, die unten mit Bezug auf die Figuren 12 bis 16 dargestellt sind.

Figur 10 zeigt eine weitere schematische axonometrische Darstellung eines oberen Bereichs des Korpus 70 aus Figur 9. Die obere Abdeckung 76 ist gewölbt und weist insbesondere die Gestalt eines Ausschnitts einer Oberfläche eines Torus auf. Die obere Abdeckung 76 ist in Figur 10 ähnlich wie in einer Explosionsdarstellung nach vertikal oben verschoben dargestellt. Die Abdeckung 76 kann mit einer Rückwand 73 des Korpus 70 mithilfe eines oder mehrerer Scharniere oder Gelenke verbunden sein. Alternativ kann die obere Abdeckung 76 auf Schiene horizontal verschiebbar oder mittels einer Hubeinrichtung vertikal anhebbar sein. Unter der oberen Abdeckung 76 ist eine Ablagefläche 77 vorgesehen, beispielsweise für medizinische Instrumente, Verbrauchsmaterial, Patientenakten oder andere Unterlagen.

Figur 11 zeigt eine weitere schematische axonometrische Darstellung des Korpus 70 aus den Figuren 9 und 10. Einzelne Bestandteile des Korpus 70 sind ähnlich einer Explosionsdarstellung vertikal und/oder horizontal gegeneinander verschoben, jedoch in der vorgesehenen räumlichen Orientierung dargestellt. Eine Bodenplatte 71 und ein Fachboden 74, der die Ablagefläche 77 (vgl. Figur 10) bildet, weisen jeweils die Gestalt eines Segments eines Kreisbogens mit zwei geraden, radial verlaufenden Randabschnitten und zwei konzentrisch kreisbogenförmigen Randabschnitten auf. Die Rückwand 73 ist - zumindest im montierten Zustand - in der Gestalt eines Ausschnitts eines Kreiszylindermantels gekrümmt. Die Seitenwände 72, 75 sind vertikal und radial orientiert.

Bodenplatte 71, Seitenwände 72, 75, Rückwand 73 und Fachboden 74 sind durch Verbinder 78 mechanisch starr verbunden. Die Verbinder 78 sind beispielsweise unter dem Markennamen CONEX19 angebotene Verbinder der Stork GmbH & Co KG, Marienfeld.

Figur 12 zeigt eine schematische Darstellung eines Schnitts entlang einer horizontalen Ebene durch das Staumöbel 60 aus Figur 9. In Figur 12 ist der Grundriss des Korpus 70 in Gestalt eines Segments eines Kreisbogens erkennbar. Gerade und radiale Ränder des Grundrisses werden durch die Seitenwände 72 gebildet. Konzentrisch-kreisbogenförmige Ränder werden durch die Frontfläche 61 und die Rückwand 73 gebildet. Wie bereits in Figur 9 erkennbar ist, muss die Frontfläche 61 keine durchgehende geschlossene Fläche sein, wie dies in den Figuren 1 bis 3 erscheint, sondern kann Öffnungen aufweisen oder teilweise oder vollständig offen sein.

In Figur 12 sind ferner die Grundrisse der in Figur 9 gezeigten und im Korpus 70 angeordneten Einsätze 81, 82, 84, 85 sichtbar. Im Unterschied zum Korpus 70 weisen die Einsätze 81, 82, 84, 85 Grundrisse mit parallelen geraden Seiten und einer zu den Seiten orthogonalen geraden Rückseite auf. Lediglich die Vorderseiten der Grundrisse der Einsätze 81, 82, 84, 85 sind kreisbogenförmig und an die Frontfläche 61 des Staumöbels angepasst bzw. bilden diese.

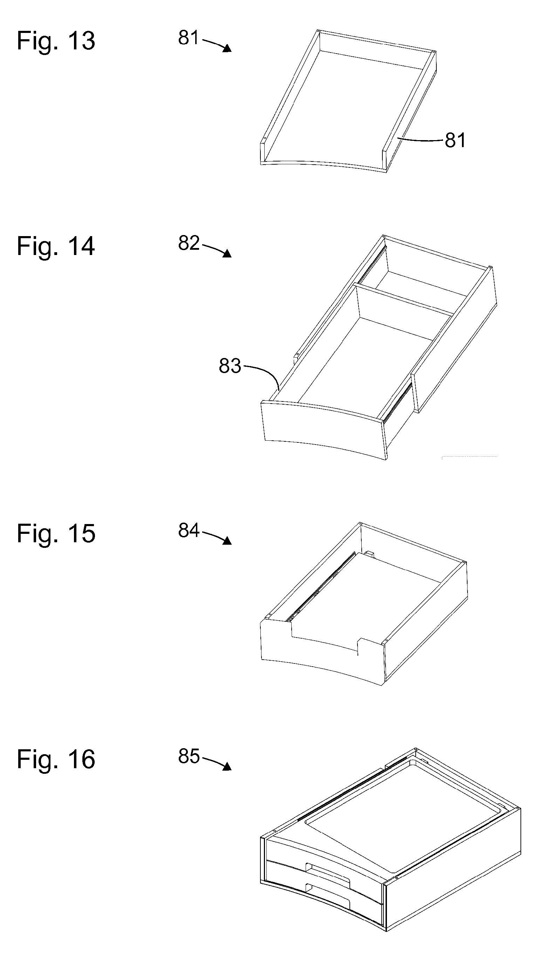

Figur 13 zeigt eine schematische axonometrische Darstellung eines ersten Einsatzes 81 in Form eines nach vorne und nach oben offenen Faches mit parallelen und ebenen Seitenwänden 86.

Figur 14 zeigt eine schematische axonometrische Darstellung eines weiteren Einsatzes in Gestalt einer Schubladeneinheit 82 mit einer ausziehbaren Schublade 83 zwischen ebenfalls parallelen und ebenen Seitenwänden.

Figur 15 zeigt eine schematische axonometrische Darstellung eines weiteren Einsatzes in Form eines Abwurfs 84 mit parallelen und ebenen Seitenwänden. Der Abwurf 84 ist vorne weitgehend, aber nicht vollständig geschlossen, um eine Entsorgung verwendeter und unsteriler Instrumente oder von verwendetem Verbrauchsmaterial in den Abwurf 84 zu ermöglichen.

Figur 16 zeigt eine schematische axonometrische Darstellung einer Besteckfacheinheit 85 mit zwei parallelen Seitenwänden 86. Die Besteckfacheinheit 85 weist zwei übereinander angeordnete Schubladen auf, die unabhängig voneinander ähnlich wie bei der Schubladeneinheit 82 aus Figur 14 nach vorne ausziehbar sind.

In Figuren 13 bis 16 ist erkennbar, dass alle Einsätze 81, 82, 84, 85 an die gekrümmte Frontfläche 61 (vgl. Figuren 1 bis 4, 12) angepasste Vorderseiten, insbesondere entsprechend gekrümmte Ränder oder Blenden aufweisen.

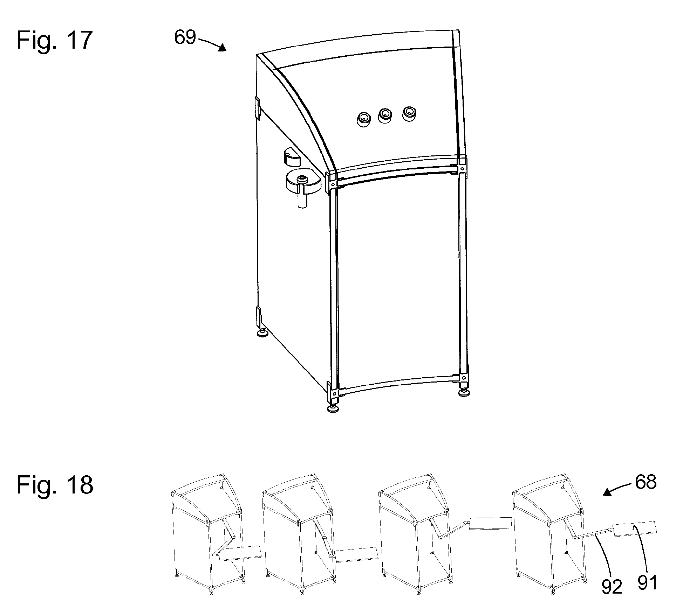

Figur 17 zeigt eine schematische axonometrische Darstellung eines schrankförmigen medizinischen Geräts 69, wie es bei dem Ausführungsbeispiel der Figuren 4 bis 6 Verwendung finden kann.

Figur 18 zeigt vier verschiedene schematische axonometrische Darstellungen eines für einen Computer optimierten Staumöbels 68. Eine Tastaturablage 91 ist über einen Tragarm 92 mit mehreren Gelenken derart mit dem Staumöbel 68 verbunden, dass die Tastaturablage 91 innerhalb weiter Grenzen frei positioniert werden kann. Vier verschiedene mögliche Positionen der Tastaturablage 91 sind in Figur 18 dargestellt.

### Bezugszeichen

- 10: medizinischer Arbeitsplatz
- 11: Raumwand
- 12: Patient
- 13: Behandlungsstuhl für Patienten 12
- 16: medizinisches Personal
- 17: Sitzplatz für medizinisches Personal 16
- 18: Symmetrieachse
- 19: Kreisbogen
- 21: erste Tragschieneneinrichtung
- 22: erster Bildschirm an der ersten Tragschiene 21
- 23: zweite Tragschieneneinrichtung
- 24: zweiter Bildschirm an der zweiten Tragschiene 23
- 25: dritte Tragschieneneinrichtung
- 26: dritter Bildschirm an der dritten Tragschiene 25
- 28: Kabelkanal in Tragschiene 21, 23, 25
- 29: Befestigungseinrichtung
- 40: Transportschieneneinrichtung
- 41: Beladebereich der Transportschieneneinrichtung 40
- 42: Verwendungsbereich der Transportschieneneinrichtung 40
- 43: Entladebereich der Transportschieneneinrichtung 40
- 48: an Transportschieneneinrichtung 40 verschiebbarer Haltarm für Instrumententräger 49
- 49: Instrumententräger an Haltearm 48
- 51: Haltearm
- 52: Instrumententräger
- 53: Haltearm
- 54: Instrumententräger
- 60: Staumöbel
- 61: Frontfläche des Staumöbels 60
- 62: Rückfläche des Staumöbels 60
- 65: weiteres Staumöbel
- 68: Staumöbel für Computer
- 69: schrankförmiges medizinisches Gerät
- 70: Korpus
- 71: Bodenplatte des Korpus 70
- 72: untere Seitenwand des Korpus 70
- 73: Rückwand des Korpus 70
- 74: Fachboden des Korpus 70
- 75: obere Seitenwand des Korpus 70
- 76: obere Abdeckung des Korpus 70
- 77: Ablagefläche unter der oberen Abdeckung 76
- 78: Verbinder
- 81: offenes Fach
- 82: Schubladeneinheit
- 83: ausziehbare Schublade der Schubladeneinheit
- 84: Abwurf
- 85: Besteckfacheinheit
- 86: Seitenwand
- 91: Tastaturablage
- 92: Tragarm für Tastaturablage 91

## Patentansprüche

1. **Medizinischer Arbeitsplatz** (10), mit: **Staumöbeln** (60) zur Aufnahme medizinischer Instrumente oder Utensilien; einer **Transportschieneneinrichtung** (40) zum Halten eines entlang der Transportschieneneinrichtung (40) verfahrbaren Instrumententrägers (49), wobei die Transportschieneneinrichtung (40) einen **Beladebereich** (41), einen **Entladebereich** (43) und zwischen dem Beladebereich (41) und dem Entladebereich (43) einen **Verwendungsbereich** (42) aufweist, wobei der Verwendungsbereich (42) in einem Raumbereich über den Staumöbeln (60) angeordnet ist wobei der Verwendungsbereich **bogenförmig** ist,
wobei die Staumöbel (60) **kreisbogenförmig** um eine für medizinisches Personal (16) vorgesehene Position (17) angeordnet sind und eine für einen **Patienten** (12) während einer medizinischen Tätigkeit vorgesehene Position (13) in dem durch die Staumöbel (60) gebildeten **Kreisbogen** (19) angeordnet ist, **dadurch gekennzeichnet dass** eine bogenförmige Tragschieneneinrichtung (21, 23, 25) zum Halten eines Geräts (22, 24, 26) vorgesehen ist, wobei das Gerät (22, 24, 26) für die Verwendung durch medizinisches Personal (16) vorgesehen und entlang der Tragschieneneinrichtung (21, 23, 25) verschiebbar ist.

2. Medizinischer Arbeitsplatz (10) nach dem vorangehenden Anspruch, bei dem die Transportschieneneinrichtung (40) in einer horizontalen Ebene über und beabstandet von den Staumöbeln (60) angeordnet ist.

3. Medizinischer Arbeitsplatz (10) nach einem der vorangehenden Ansprüche, bei dem zumindest entweder der Beladebereich (41) oder der Entladebereich (43) einen **größeren Abstand** von einer für medizinisches Personal (16) vorgesehenen Position (17) aufweist als der Verwendungsbereich (42).

4. Medizinischer Arbeitsplatz (10) nach dem vorangehenden Anspruch, bei dem die Tragschieneneinrichtung (21, 23, 25) zumindest einen **kreisbogenförmigen** Abschnitt mit einem Mittelpunkt an oder über der für medizinisches Personal (16) vorgesehenen Position (17) aufweist.

5. Medizinischer Arbeitsplatz (10) nach dem Anspruch 1, bei dem die Tragschieneneinrichtung (21, 23, 25) einen **Kanal** (28) zur Aufnahme eines Kabels oder Schlauchs aufweist.

6. Medizinischer Arbeitsplatz (10) nach dem vorangehenden Anspruch, bei dem die Tragschieneneinrichtung (21, 23, 25) einen nach oben offenen U-förmigen Querschnitt aufweist, wobei das Innere des Querschnitts der Tragschieneneinrichtung (21, 23, 25) den Kanal (28) bildet.

7. Medizinischer Arbeitsplatz (10) nach einem der vorangehenden Ansprüche, bei dem mindestens ein, insbesondere mehrere oder alle, Staumöbel (60) einen Korpus (70) mit einer Bodenplatte (71) in der Form eines Segments eines Kreisrings und nicht-parallelen Seitenwänden (72, 75) und in dem Korpus (70) einen Einsatz (81, 82, 84, 85) zum Aufnehmen eines Gegenstands aufweist, wobei der Einsatz (81, 82, 84, 85) parallele Seitenwände (86) aufweist.

8. Medizinischer Arbeitsplatz (10) nach dem vorangehenden Anspruch, bei dem der Einsatz (82) einen Auszug (83) umfasst

## Claims

1. Medical workstation (10) with: storage units (60) for receiving medical instruments or tools; a transport rail (40) for holding an instrument carrier (49) that is movable along the transport rail (40), wherein the transport rail (40) has a loading area (41), an unloading area (43) and, between the loading area (41) and the unloading area (43), a use area (42), wherein the use area (42) is arranged in a room area above the storage units (60), wherein the use area is arc-shaped, wherein the storage units (60) are arranged in the shape of an arc of a circle around a position (17) provided for medical personnel (16) and a position (13) provided for a patient (12) during a medical activity is arranged in the arc of a circle (19) formed by the storage units (60), **characterized in that** an arc-shaped support rail (21, 23, 25) for holding an appliance (22, 24, 26) is provided, wherein the appliance (22, 24, 26) is provided for use by medical personnel (16) and is movable along the support rail (21, 23, 25).

2. Medical workstation (10) according to the preceding claim, in which the transport rail (40) is arranged in a horizontal plane above and at a distance from the storage units (60).

3. Medical workstation (10) according to one of the preceding claims, in which at least either the loading area (41) or the unloading area (43) is at a greater distance from a position (17) provided for medical personnel (16) than is the use area (42).

4. Medical workstation (10) according to the preceding claim, in which the support rail (21, 23, 25) has at least one portion in the shape of an arc of a circle with a center point at or over the position (17) provided for medical personnel (16).

5. Medical workstation (10) according to Claim 1, in which the support rail (21, 23, 25) has a channel (28) for receiving a cable or hose.

6. Medical workstation (10) according to the preceding claim, in which the support rail (21, 23, 25) has an upwardly open U-shaped cross section, wherein the interior of the cross section of the support rail (21, 23, 25) forms the channel (28).

7. Medical workstation (10) according to one of the preceding claims, in which at least one storage unit (60), in particular several storage units (60) or all storage units (60), has/have a trunk (70) with a bottom plate (71) in the form of a segment of a circular ring and non-parallel side walls (72, 75) and, in the trunk (70), a drawer unit (81, 82, 84, 85) for receiving an object, wherein the drawer unit (81, 82, 84, 85) has parallel side walls (86).

8. Medical workstation (10) according to the preceding claim, in which the drawer unit (82) comprises a pull-out drawer (83).

## Revendications

1. Poste de travail médical (10), comprenant : des meubles de rangement (60) pour recevoir des instruments ou ustensiles médicaux ; un dispositif de rail de transport (40) pour supporter un porte-instruments (49) déplaçable le long du dispositif de rail de transport (40), le dispositif de rail de transport (40) présentant une région de chargement (41), une région de déchargement (43) et une région d'utilisation (42) entre la région de chargement (41) et la région de déchargement (43), la région d'utilisation (42) étant disposée dans une région de l'espace au-dessus des meubles de rangement (60), la région d'utilisation étant de forme courbe, les meubles de rangement (60) étant disposés sous forme d'arc de cercle autour d'une position (17) prévue pour le personnel médical (16) et une position (13) prévue pour un patient (12) pendant une activité médicale étant disposée dans l'arc de cercle (19) formé par les meubles de rangement (60), **caractérisé en ce qu'**un dispositif de rail de support de forme courbe (21, 23, 25) est prévu pour supporter un appareil (22, 24, 26), l'appareil (22, 24, 26) étant prévu pour l'utilisation par le personnel médical (16) et pouvant être déplacé le long du dispositif de rail de support (21, 23, 25).

2. Poste de travail médical (10) selon la revendication précédente, dans lequel le dispositif de rail de transport (40) est disposé dans un plan horizontal au-dessus et à distance des meubles de rangement (60).

3. Poste de travail médical (10) selon l'une quelconque des revendications précédentes, dans lequel au moins soit la région de chargement (41) soit la région de déchargement (43) présente une plus grande distance par rapport à une position (17) prévue pour le personnel médical (16) que la région d'utilisation (42).

4. Poste de travail médical (10) selon la revendication précédente, dans lequel le dispositif de rail de support (21, 23, 25) présente au moins une portion en forme d'arc de cercle avec un centre au niveau de ou au-dessus de la position (17) prévue pour le personnel médical (16).

5. Poste de travail médical (10) selon la revendication 1, dans lequel le dispositif de rail de support (21, 23, 25) présente un canal (28) pour recevoir un câble ou un flexible.

6. Poste de travail médical (10) selon la revendication précédente, dans lequel le dispositif de rail de support (21, 23, 25) présente une section transversale en forme de U ouverte vers le haut, l'intérieur de la section transversale du dispositif de rail de support (21, 23, 25) formant le canal (28).

7. Poste de travail médical (10) selon l'une quelconque des revendications précédentes, dans lequel au moins un, en particulier plusieurs ou la totalité des meubles de rangement (60) présentent un corps (70) avec une plaque de fond (71) en forme de segment de bague circulaire et des parois latérales non parallèles (72, 75) et, dans le corps (70), un insert (81, 82, 84, 85) pour recevoir un objet, l'insert (81, 82, 84, 85) présentant des parois latérales parallèles (86).

8. Poste de travail médical (10) selon la revendication précédente, dans lequel l'insert (82) comprend une rallonge (83).
